# EUROPEAN PATENT SPECIFICATION

(11) **EP 1 623 718 B1**
(45) Date of publication and mention of the grant of the patent: **05.01.2011**
(21) Application number: 03721059.8
(22) Date of filing: 07.05.2003
(51) Int. Cl.: A61K 8/64, A23L 1/305, A61Q 19/08, A61Q 19/00

(54) **SKIN COLLAGEN PRODUCTION PROMOTER**
MITTEL ZUR FÖRDERUNG DER HAUTKOLLAGENPRODUKTION
PROMOTEUR DE LA PRODUCTION DU COLLAGENE DE LA PEAU

(43) Date of publication of application: 08.02.2006
(73) Proprietor: Snow Brand Milk Products Co., Ltd., Sapporo-shi Hokkaido 065-0043 (JP)
(72) Inventor: MORITA, Yoshikazu, Kawagoe-shi, Saitama 350-1167 (JP); TAKADA, Yukihiro, Kawagoe-shi, Saitama 350-0811 (JP); TOBA, Yasuhiro, Musashino-shi, Tokyo 180-0003 (JP); OOI, Wataru, Kawagoe-shi, Saitama 350-1167 (JP); KAWAKAMI, Hiroshi, Kawagoe-shi, Saitama 350-1142 (JP)
(74) Representative: Wakerley, Helen Rachael
(86) International application number: PCT/JP2003/005707
(87) International publication number: WO 2004/098632

(56) References cited:
- EP-A1- 0 556 083
- EP-A1- 1 010 430
- EP-A2- 0 704 218
- WO-A1-02/05828
- WO-A1-03/006500
- WO-A1-03/015724
- CA-A1- 2 296 311
- JP-A- 3 020 206
- JP-A- 8 133 943
- JP-A- 9 087 164
- JP-A- 9 187 250
- JP-A- 2003 144 095
- US-A- 5 976 597
- US-B1- 6 506 732
- MATSUOKA Y. ET AL.: 'Cystatin C in milk basic protein (MPB) and its inhibitory effect on bone resorption in vitro' BIOSCI. BIOTECHNOL. BIOCHEM. vol. 66, no. 12, 2002, pages 2531 - 2536, XP001176746
- KATO K. ET AL.: 'Milk basic protein enhances the bone strenght in ovariectomized rats' J. FOOD BIOTECHEM. vol. 24, no. 6, 2000, pages 467 - 476, XP002971512
- TAKADA Y. ET AL.: 'Hone no kenko ni kiyo suru atarashii chichi no chikara: nyuenki-sei tanpaku-shitsu (MPB)' FINE CHEMICAL vol. 31, no. 3, 2002, pages 5 - 13, XP002983572
- YAMAMURA J. ET AL.: 'Gyunyu ni fukumareru nyuenki-sei tanpaku-shitsu (MBP) no aratana kotsutaisha kaizen sayo no hakken' BRAIN TECHNO NEWS vol. 87, 2001, pages 24 - 28, XP002983573
- TOBA Y. ET AL.: 'Nyuenki-sei tanpaku-shitsu (MBP):hone no kenko o sasaeru gyunyuchu no shinkina kino seibun' BIO CLINICA vol. 16, no. 13, 2001, pages 1227 - 1230, XP002983574

## Description

### [Technical Field]

The present invention relates to a cosmetic for promoting skin collagen production, which are useful in preventing skin chapping, wrinkles, worsening in skin fitness, or the like.

### [Background Art]

In recent years, studies on the mechanism of skin have been carried out, and as a result, it has been confirmed that macroscopic causes of skin dry feeling and skin chapping are intricately involved in effects of sunlight (ultraviolet ray), dryness, oxidization, or the like in addition to effects due to disturbance of metabolism with age. It has been found that effects caused by such factors significantly decrease collagen fiber that is the most principal matrix component in the corium. When a mechanism to keep tension such as skin fitness or elasticity that is maintained by collagen fiber is destroyed by an effect of ultraviolet ray or the like, wrinkles or sags of the skin are increased. Meanwhile, a molecule of collagen may maintain water, so that it helps maintaining skin moisture. Therefore, when collagen is destroyed by external factors, the skin becomes dry and rough.

Because of the aforementioned facts, there has been desired a skin collagen production promoter that is harmless and may prevent wrinkles or sags of skin by promoting biosynthesis of collagen that is one of the major components of the corium layer.

### [Disclosure of the Invention]

For solving such problems, the inventors of the present invention have made extensive studies on a substance that exerts a promoting effect on skin collagen production that is widely contained in food materials, and as a result, they have found out that a milk-derived basic protein fraction obtained by adsorbing a basic protein of contacting milk or a milk-derived material with a catoon exchange resin and then elleting the fraction adsorbed to the resin width an element having a salt concentration of 0.1 to 1 M;and/ or a basic peptide fraction obtained by degradation of the milk-derived basic protein fraction with a protease such as pepsin or pancreatin may increase the amount of skin collagen. Moreover, they have found out that the milk-derived basic protein fraction or the basic peptide fraction may be used as an active ingredient of a skin collagen production promoter.

Therefore, the present invention provides the use of the milk-derived basic protein fraction and/or the basic peptide fraction as a skin collagen production promoter in the form of a cosmetic.

The present invention is set out in the accompanying claims. There is provided, a cosmetic method of treating the skin to prevent or treat skin wrinkles, skin sags or skin chapping comprising the administration of a composition comprising a milk-derived basic protein fraction obtained by adsorbing a basic protein by contacting milk or a milk-derived material with a cation exchange resin and then eluting the fraction adsorbed to the resin with an eluent having a salt concentration of 0.1 to 1 M.

In a further aspect, there is provided a cosmetic method of treating the skin to prevent or treat skin wrinkles, skin sags or skin chapping comprising the administration of a composition comprising a milk-derived basic peptide fraction obtained by i) adsorbing a basic protein by contacting milk or a milk-derived material with a cation exchange resin and then eluting the fraction adsorbed to the resin with an eluent having a salt concentration of 0.1 to 1 M to obtain a basic protein fraction, and ii) degrading the said basic protein with a protease.

The "skin collagen production promoter" refers to a substance that exerts a promoting effect on skin collagen production in the case of oral administration, application, or the like. Meanwhile, the "food or beverage product for promoting skin collagen production" refers to, among the skin collagen production promoters, a substance that is intended to be orally administered after being formulated into a powder, granule, tablet, capsule, drink, or the like, or administered after being formulated and incorporated into a nutritional supplement, food or beverage product, or the like. Meanwhile, the "cosmetic for promoting skin collagen production" refers to, among the skin collagen production promoters, a substance to be applied to skin after being formulated into an ointment, gel, cream, spray, patch, lotion, or the like. Moreover, the above-described food or beverage product for promoting skin collagen production includes a substance that is orally administered after being formulated without additional treatment, for the sake of convenience, while the above-described cosmetic for promoting skin collagen production includes a substance that is administered to skin after being formulated, for the sake of convenience.

The composition used in the methods of the present invention are characterized by including, as an active ingredient, a milk-derived basic protein fraction and/or a basic peptide fraction obtained by degradation of the milk-derived basic protein fraction with a protease wherein the milk-derived basic protein fraction is obtained from mammal milk such as bovine milk, humanmilk, goatmilk, or ewemilk, while the basic peptide fraction may be obtained by a reaction of a protease with a milk-derived basic protein fraction.

The milk-derived basic protein fraction has the following properties as shown in Test Examples 1 to 3 below.
1) The fraction includes several kinds of proteins each having a molecular weight in the range of 3,000 to 80,000, which is a result of determination by sodium dodecyl sulfate-polyacrylamide gel electrophoresis (SDS-PAGE).
2) The fraction includes proteins at a rate of 95% by weight or more.
3) The proteins mainly include lactoferrin and lactoperoxidase.
4) The amino acid composition of the proteins includes basic amino acids such as lysine, histidine, and arginine each at a rate of 15% by weight or more.

The basic protein fraction is obtained as follows: a milk-derived material such as skim milk or whey is brought into contact with a cation exchange resin to adsorb basic proteins, a basic protein fraction adsorbed to the resin is eluted with an eluent having a salt concentration of 0.1 to 1 M, the eluted fraction is collected, and desalting and concentration are performed by a reverse osmosis (RO) membrane, electrodialysis (ED) method, or the like, followed by drying, if necessary.

Meanwhile, known examples of a method of obtaining a milk-derived basic protein fraction, a method of obtaining the fraction by contacting milk or a milk-derived material with a cation exchanger to adsorb basic proteins and then eluting the basic protein fraction adsorbed to the cation exchanger with an eluent having a pH of more than 5 and an ionic strength of more than 0.5 (JP-A-05-202098), a method of obtaining the fraction using an alginic acid gel (JP-A-61-24 6198), a method of obtaining the fraction from whey using inorganic porous particles (JP-A-01-086839), and a method of obtaining the fraction from milk using a sulfated ester compound (JP-A-63-255300). The basic protein fractions obtained by such methods may be used in the present disclosure.

In one distance of the present disclosure, the milk-derived basic peptide fraction has the same amino acid composition as that of the basic protein fraction. For example, a protease such as pepsin, trypsin, chymotrypsin, or pancreatin is allowed to react with the milk-derived basic protein fraction obtained by one of the above-described methods, to thereby obtain a basic peptide composition having an average molecular weight of 4,000 or less. The lower limit of the molecular weight is preferably 500 or more.

When the skin collagen production promoter is orally administered or applied, it exerts a promoting effect on skin collagen production. In the case of oral administration of the skin collagen production promoter, the milk-derived basic protein fraction or basic peptide fraction (obtained as discussed above) serving as an active ingredient may be used after it is formulated into a powder, granule, tablet, capsule, drink, or the like according to the conventional method. An oral agent such as a powder, granule, tablet, or capsule is formulated by using, for example, starch, lactose, sucrose, mannitol, carboxymethylcellulose, corn starch, inorganic salts, or the like according to the conventional method. For this kind of formulation, there may be used, in addition to the above described vehicles, a binder, disintegrator, surfactant, lubricant, fluidity promoter, colorant, flavor, or the like. More specifically, examples of the binder include starch, dextrin, powdered acacia, gelatin, hydroxypropyl starch, sodium carboxymethylcellulose, methylcellulose, ethylcellulose, crystalline cellulose, and polyvinylpyrrolidone. Meanwhile, examples of the disintegrator include starch, hydroxypropyl starch, carboxymethylcellulose, sodium carboxymethylcellulose, cross-linked sodium carboxymethylcellulose, and crystalline cellulose. Examples of the surfactant include soybean lecithin and sucrose fatty acid ester. Examples of the lubricant include talc, wax, sucrose fatty acid ester, and hydrogenated vegetable oil. Examples of the fluidity promoter include silicic anhydride, dried aluminum hydroxide, and magnesium silicate.

Moreover, the milk-derived basic protein fraction or the basic peptide fraction may be incorporated in a nutritional supplement, a food or beverage product, or the like, after being formulated. In addition, when the basic protein fraction or the basic peptide fraction is incorporated in combination with an ingredient such as vitamin C that is conventionally considered to have an effect on collagen production, further promoting effect on skin collagen production may be expected. Note that the milk-derived basic protein fraction or the basic peptide fraction is relatively stable to heat, so that a material containing a milk-derived basic protein fraction or basic peptide fraction may be sterilized by heating under a general condition.

In the case of application of the skin collagen production promoter, the promoter may be incorporated in a known ingredient that is generally used depending on the intended use, to thereby prepare various dosage forms such as a liquid formulation, solid formulation, and semi-solid formulation. Examples of a preferable composition include an ointment, gel, cream, spray, patch, lotion, and powder. For example, the skin collagen production promoter may be mixed with: a hydrocarbon such as petrolatum; higher fatty acid lower alkyl ester such as stearyl alcohol or isopropyl myristate; animal oil and fat such as lanoline; polyalcohol such as glycerin; surfactant such as glycerin fatty acid ester or polyethyleneglycol monostearate; inorganic salt; wax; resin; water; and if necessary, preservative such as methyl paraoxybenzoate or butyl paraoxybenzoate, to thereby produce a cosmetic product for promoting skin collagen production.

The effective amount of the skin collagen production promoter for oral administration is appropriately defined depending on the promoter's dosage form, administration method, intended use, and the age, weight, and symptom of a subject to be applied, and it is not constant. However, the results of animal experiments using rats revealed that, in order to exert a promoting effect on skin collagen production, a milk-derived basic protein fraction and/or basic peptide fraction must be taken in the amount of at least 20 mg/kg weight of a rat . Therefore, according to the extrapolation method, when at least one of a basic protein fraction and/or a basic peptide fraction is taken in the amount of at least 20 mg/day/adult, usually, the effect may be expected. Accordingly, the fraction may be incorporated in a food or beverage product so as to achieve the requirement . Note that administration may be performed several times a day, if necessary.

The effective amount of the skin collagen production promoter for application varies depending on the promoter's dosage form, but the basic protein fraction or basic peptide fraction may preferably be incorporated in the amount of 0.001 to 2% by weight on the basis of the amount of all compositions to be applied. However, the incorporation amount of a product to be diluted when used such as a bath powder may further be increased.

Next, the present disclosure, comprising the invention, will be described in detail with reference to examples and test examples.

### [Example 1]

A column (diameter 5 cm × height 30 cm) filled with 400 g of sulfonated Chitopearl (manufactured by Fuji Spinning Co., Ltd.) as a cation exchange resin was thoroughly washed with deionized water, and then 40 L of unsterilized skim milk (pH 6. 7) was allowed to pass through the column at a flow rate of 25 ml/min. After the passing, the column was thoroughly washed with deionized water, and a basic protein fraction adsorbed to the resin was eluted with 0.02 M carbonate buffer (pH 7.0) containing 0.98 M sodium chloride. Thereafter, the eluted solution was desalted and concentrated by using a reverse osmosis (RO) membrane, and then freeze-drying was performed, to thereby obtain 21 g of a powdery basic protein fraction. The thus-obtained basic protein fraction may be used without treatment as a skin collagen production promoter.

### [Test Example 1]

For the basic protein fraction obtained in Example 1, the molecular weights were determined by sodium dodecyl sulfate-polyacrylamide gel electrophoresis (SDS-PAGE). As a result, they were distributed in the range of 3,000 to 80,000.

### [Test Example 2]

The basic protein fraction obtained in Example 1 was analyzed for ingredient composition. The results are shown in Table 1. As shown in the table, most ingredients in the fraction were found to be proteins.

**Table 1**

| | |
|---|---|
| Water | 1.06 (wt%) |
| Proteins | 96.50 |
| Lipids | 0.56 |
| Ash | 0.27 |
| Others | 1.61 |

### [Test Example 3]

The basic protein fraction obtained in Example 1 was hydrolyzed with 6 N hydrochloric acid at 110 °C for 24 hours, and then analyzed for amino acid composition by using an apparatus for amino acid analysis (type L-8500, manufactured by Hitachi, Ltd.). The results are shown in Table 2. The basic protein fraction has an amino acid composition containing 15% by weight or more of basic amino acids.

**Table 2**

| | |
|---|---|
| Aspartic acid | 10.1 (wt%) |
| Serine | 5.3 |
| Glutamic acid | 12.3 |
| Proline | 4.7 |
| Alanine | 5.7 |
| Leucine | 10.2 |
| Lysine | 8.4 |
| Histidine | 2.5 |
| Arginine | 7.2 |
| Others | 33.6 |

### [Example 2]

A column (diameter 100 cm × height 10 cm) filled with 30 kg of SP Toyopearl (manufactured by Tosoh Corporation) as a cation exchange resin was thoroughly washed with deionized water, and then 3 t of cheese whey (pH 6.2) that had been sterilized by heating at 121°C for 30 seconds was allowed to pass through the column at a flow rate of 10 L/min. After the passing, the column was thoroughly washed with deionized water, and a basic protein fraction adsorbed to the resin was eluted with 0.1 M citrate buffer (pH 5.7) containing 0. 9 M sodium chloride. Thereafter, the eluted solution was desalted and concentrated by the electrodialysis (ED) method, and then freeze-drying was performed, to thereby obtain 183 g of a powdery basic protein fraction. The thus-obtained basic protein fraction may be used without treatment as a skin collagen production promoter.

### [Example 3]

50 g of the basic protein fraction obtained in Example 2 was dissolved in 10 L of distilled water, and then Pancreatin (manufactured by Sigma) was added thereto at a concentration of 1%, followed by reaction at 37°C for 2 hours. After the reaction, heat treatment was performed at 80°C for 10 minutes to inactivate enzymes, and concentration and freeze-drying were performed, to thereby obtain 48.3 g of a powdery basic peptide fraction. The thus-obtained basic peptide fraction may be used without treatment as a skin collagen production promoter.

### [Test Example 4]

For the basic protein fraction obtained in Example 2 and the basic peptide fraction obtained in Example 3, animal experiments were performed by using rats to investigate the promoting effect on skin collagen production. 7-week-old Wistar male rats were divided into 5 test groups (n = 6) : the group to which physiological saline was administered (group A) ; the group to which the basic protein fraction obtained in Example 2 was administered at a concentration of 20 mg/kg weight of a rat (group B); the group to which the basic protein fraction obtained in Example 2 was administered at a concentration of 200 mg/kg weight of a rat (group C) ; the group to which the basic peptide fraction obtained in Example 3 was administered at a concentration of 20 mg/kg weight of a rat (group D); and the group to which the basic peptide fraction obtained in Example 3 was administered at a concentration of 200 mg/kg weight of a rat (group E). The rats were bred for 10 weeks while each fraction was administered by a sonde once a day.

For the amount of skin collagen, the corium of each rat was treated in accordance with the method by Nimni et al. (see Arch. Biochem. Biophys., p. 292, 1967), and then the amount of hydroxyproline in the soluble fraction was determined. Hydroxyproline is a special kind of amino acid contained only in collagen and accounts for about 10% of all amino acids that constitute collagen, so that the amount of collagen may be estimated (see Ryuji Asano et al., Bio Industry, p. 12, 2001). The results are shown in Table 3.

**Table 3**

| Group | Hydroxyproline amount (µg/ml) |
|---|---|
| Group A | 0.35±0.03^{a} |
| Group B | 0.72±0.07^{bc} |
| Group C | 0.91±0.9^{d} |
| Group D | 0.63±0.06^{b} |
| Group E | 0.84±0.08^{cd} |

| | |
|---|---|
| The values represent mean ± standard deviation (n = 6). Meanwhile, there is a significant difference between different alphabets (p < 0.05). | |

According to the results, it was found that the amounts of hydroxyproline in the soluble fractions of groups B, C, D, and E obtained after 10 weeks were significantly higher than that of group A.

The results revealed that the basic protein fraction and the basic peptide fraction each have the promoting effect on skin collagen production and are effective as skin collagen production promoters.

Moreover, it was found that the promoting effect on skin collagen production is exerted in the case that the basic protein fraction or the basic peptide fraction is administered at a concentration of at least 20 mg/kg weight of a rat.

### [Example 4]

A beverage for promoting skin collagen production having the composition shown in Table 4 was produced according to the conventional method. The produced beverage had a good flavor, the flavor did not deteriorate even after preservation at ordinary temperature for 1 year, and there was no problem such as generation of precipitates.

**Table 4**

| | |
|---|---|
| Mixed isomerized sugar | 15.0 (wt%) |
| Fruit juice | 10.0 |
| Citric acid | 0.5 |
| Basic protein fraction powder (Example 2) | 0.1 |
| Flavor | 0.1 |
| Mineral mixture | 0.1 |
| Water | 74.2 |

### [Example 5]

Dough having the composition shown in Table 5 was prepared according to the conventional method, molded, and baked to produce biscuits for promoting skin collagen production.

**Table 5**

| | |
|---|---|
| Flour | 50.0 (wt%) |
| Sugar | 20.0 |
| Salt | 0.5 |
| Margarine | 12.5 |
| Egg | 12.5 |
| Water | 2.5 |
| Mineral mixture | 0.8 |
| Basic protein fraction powder (Example 2) | 1.2 |

### [Example 6]

A skin collagen production promoter having the composition shown in table 6 was produced according to the conventional method.

**Table 6**

| | |
|---|---|
| Crystallized dextrose monohydrate | 83.5 (wt%) |
| Basic protein fraction powder (Example 2) | 10.0 |
| Mineral mixture | 5.0 |
| Sugar ester | 1.0 |
| Flavor | 0.5 |

### [Test Example 5]

For the basic protein fraction obtained in Example 2 and the basic peptide fraction obtained in Example 3, the promoting effect on skin collagen production was investigated by an experiment using a normal human fibroblast strain[CCD45SK (ATCCRL 1506) collected from the skin of a white female] . Modified Eagle' s medium (MEM, 10-101, manufactured by Dainippon Pharmaceutical Co., Ltd.) containing 10% by volume of bovine fetal serum (hereinafter abbreviated as FBS) was used to inoculate a normal human fibroblast strain to a 24-well plate at a concentration of 4 × 10⁴ cells/well/0.4 ml. Then, culture was performed under 5% carbon dioxide and saturated water vapor at 37°C for 24 hours, and the medium was exchanged for modified Eagle' s medium containing 0.6% by volume of FBS. Thereafter, the basic protein fraction obtained in Example 2 and the basic peptide fraction obtained in Example 3 were added to each well at a concentration of 0.1% by volume, and culture was performed for 24 hours. Then, β-aminopropionitrile and tritium-L-proline were added at concentrations of 50 µg/ml and 1 µCi/ml, respectively, and culture was performed for additional 24 hours to obtain a culture medium. From the thus-obtained culture medium, a collagen fraction was fractionated according to the method by Webster et al. (see Analytical Biochemistry, p. 220, 1979), and radioactivity that had been taken in the collagen fraction was determined. Note that, as a control, a similar test was performed without addition of the basic protein fraction and the basic peptide fraction. The results are shown in Table 7.

**Table 7**

| | Collagen production (%) |
|---|---|
| Control | 100±2.1^{a} |
| Basic protein fraction powder (Example 2) | 212±4.1^{c} |
| Basic peptide fraction powder (Example 3) | 196±3.2^{b} |

| | |
|---|---|
| The values represent mean ± standard deviation (n = 6). Meanwhile, there is a significant difference between different alphabets (p < 0.05). | |

According to the results, it was found that the group to which the basic protein fraction and the basic peptide fraction were added exerted about a double ability to promote skin collagen production compared with the group to which the basic protein fraction and the basic peptide fraction were not added (control) .

The results revealed that the basic protein fraction and the basic peptide fraction affect skin fibroblasts and have the promoting effects on skin collagen production, and it was suggested that the fractions are useful as skin collagen production promoters.

### [Example 7]

A lotion having the composition shown in Table 8 was produced according to the conventional method.

**Table 8**

| | |
|---|---|
| Glycerin | 3 (wt%) |
| 1,3-butylene glycol | 3 |
| Polyoxyethylenesorbitan monooleate (20E.O.) | 0.5 |
| Methyl paraoxybenzoate | 0.15 |
| Citric acid | 0.1 |
| Sodium citrate | 1 |
| Flavor | 0.05 |
| Basic protein fraction powder (Example 2) | 0.05 |
| Purified water | 92.15 |

### [Example 8]

A cream having the composition shown in Table 9 was produced according to the conventional method.

**Table 9**

| | |
|---|---|
| Liquid paraffin | 5 (wt%) |
| White beeswax | 4 |
| Cetanol | 3 |
| Squalane | 10 |
| Lanolin | 2 |
| Stearic acid | 1 |
| Polyoxyethylenesorbitan monooleate (20E.O.) | 1.5 |
| Glyceryl monostearate | 3 |
| 1,3-butylene glycol | 6 |
| Methyl paraoxybenzoate | 1.5 |
| Flavor | 0.1 |
| Basic protein fraction powder (Example 2) | 0.5 |
| Purified water | 62.4 |

### [Test Example 6]

A practical use test was performed by using the lotion obtained in Example 7 and the cream obtained in Example 8. As a lotion and cream for comparison, there were used lotion and cream having the same compositions as those in Examples 7 and 8 except that the basic protein fraction was removed.

20 adult females having faces with sags or fine wrinkles and having dry skins were divided at random into 2 groups each having 10 subjects (groups A and B), while 20 adult females having hand chapping were divided at random into 2 groups each having 10 subjects (groups C and D). The lotion of the present invention, the lotion for comparison, the cream of the present invention, and the cream for comparison were applied to the faces of group A, the faces of group B, the hands and fingers of group C, and the hands and fingers of group D, respectively, in the same way as usual twice a day for 10 days. As a result, it was demonstrated that the lotion of the present invention significantly improved dry feeling and skin chapping compared with the lotion for comparison and had an excellent promoting effect on skin collagen production. Meanwhile, it was also found that the cream of the present invention significantly improved dry feeling and skin chapping compared with the cream for comparison and had a suppressing effect on natural exacerbation such as skin chapping.

The present disclosure provides a cosmetic method for promoting skin collagen production, using a milk-derived basic protein fraction and/or basic peptide fraction as an active ingredient. This is useful in preventing or treating skin wrinkles, sags, dry feeling, and skin chapping in a cosmetic.

## Claims

1. Use of
i) a milk-derived basic protein fraction obtained by adsorbing a basic protein by contacting milk or a milk-derivedmaterial with a cation exchange resin and then eluting the fraction adsorbed to the resin with an eluent having a salt concentration of 0.1 to 1 M; and/or
ii) a basic peptide fraction obtained by degradation of the milk-derived basic protein fraction with a protease
as a skin collagen production promoter in the form of a cosmetic.

2. Use according to claim 1, wherein the milk-derived basic protein fraction is a fraction whose amino acid composition contains 15% by weight or more of a basic amino acid.

3. Use according to claim 1 or 2, wherein the skin collagen production promoter is for the prevention or treatment of skin wrinkles, skin sags, dry feeling of the skin or skin chapping.

4. Use of
i) a milk-derived basic protein fraction obtained by adsorbing a basic protein by contacting milk or a milk-derived material with a cation exchange resin; and then eluting the fraction adsorbed to the resin with an eluent having a salt concentration of 0.1 to 1 M and/or
ii) a basic peptide fraction obtained by degradation of the milk-derived basic protein fraction with a protease
as a cosmetic skin collagen production promoter in the form of a food or beverage product.

5. A cosmetic method of treating the skin to prevent or treat skin wrinkles, skin sags or skin chapping comprising the administration of a composition comprising a milk-derived basic protein fraction obtained by adsorbing a basic protein by contacting milk or a milk-derived material with a cation exchange resin and then eluting the fraction adsorbed to the resin with an eluent having a salt concentration of 0.1 to 1 M.

6. A cosmetic method of treating the skin to prevent or treat skin wrinkles, skin sags or skin chapping comprising the administration of a composition comprising a milk-derived basic peptide fraction obtained by i) adsorbing a basic protein by contacting milk or a milk-derived material with a cation exchange resin and then eluting the fraction adsorbed to the resin with an eluent having a salt concentration of 0.1 to 1 M to obtain a basic protein fraction, and ii) degrading the said basic protein with a protease.

7. A method according to claim 5 or 6 in which the composition is in the form of a cosmetic to be applied to the skin.

8. A method according to claim 5 or 6 in which the composition is in the form of a food or beverage.

## Patentansprüche

1. Verwendung von
i) einer von Milch abgeleiteten basischen Proteinfraktion, erhalten durch Adsorbieren eines basischen Proteins durch Inkontaktbringen von Milch oder einem von Milch abgeleiteten Material mit einem Kationenaustauschharz und dann Eluieren der am Harz adsorbierten Fraktion mit einem Elutionsmittel mit einer Salzkonzentration von 0,1 bis 1 M; und/oder
ii) einer basischen Peptidfraktion, die durch den Abbau der von Milch abgeleiteten basischen Proteinfraktion mit einer Protease erhalten wird,
als Hautkollagen-Produktionspromotor in Form eines Kosmetikums.

2. Verwendung nach Anspruch 1, wobei die von Milch abgeleitete basische Proteinfraktion eine Fraktion ist, deren Aminosäurezusammensetzung 15 Gew.-% oder mehr einer basischen Aminosäure enthält.

3. Verwendung nach Anspruch 1 oder 2, wobei der Hautkollagen-Produktionspromotor für die Verhütung oder Behandlung von Hautfalten, schlaffer Haut, Trockenheitsgefühl der Haut oder spröder Haut vorgesehen ist.

4. Verwendung von
i) einer von Milch abgeleiteten basischen Proteinfraktion, erhalten durch Adsorbieren eines basischen Proteins durch Inkontaktbringen von Milch oder einem von Milch abgeleiteten Material mit einem Kationenaustauschharz und dann Eluieren der am Harz adsorbierten Fraktion mit einem Elutionsmittel mit einer Salzkonzentration von 0,1 bis 1 M; und/oder
ii) einer basischen Peptidfraktion, die durch den Abbau der von Milch abgeleiteten basischen Proteinfraktion mit einer Protease erhalten wird,
als kosmetischer Hautkollagen-Produktionspromotor in Form eines Nahrungsmittel- oder Getränkeprodukts.

5. Kosmetisches Verfahren zur Behandlung der Haut zur Verhütung oder Behandlung von Hautfalten, schlaffer Haut oder spröder Haut, das das Verabreichen einer Zusammensetzung beinhaltet, die eine von Milch abgeleitete basische Proteinfraktion umfasst, die durch Adsorbieren eines basischen Proteins durch Inkontaktbringen von Milch oder einem von Milch abgeleiteten Material mit einem Kationenaustauschharz und dann Eluieren der am Harz adsorbierten Fraktion mit einem Elutionsmittel mit einer Salzkonzentration von 0,1 bis 1 M erhalten wird.

6. Kosmetisches Verfahren zur Behandlung von Haut zur Verhütung oder Behandlung von Hautfalten, schlaffer Haut oder spröder Haut, das das Verabreichen einer Zusammensetzung umfasst, die eine von Milch abgeleitete basische Peptidfraktion umfasst, die erhalten wird durch i) Adsorbieren eines basischen Proteins durch Inkontaktbringen von Milch oder einem von Milch abgeleiteten Material mit einem Kationenaustauschharz und dann Eluieren der am Harz adsorbierten Fraktion mit einem Elutionsmittel mit einer Salzkonzentration von 0,1 bis 1 M, um eine basische Proteinfraktion zu erhalten, und ii) Abbauen des genannten basischen Proteins mit einer Protease.

7. Verfahren nach Anspruch 5 oder 6, wobei die Zusammensetzung in Form eines Kosmetikums vorliegt, das auf die Haut aufgetragen wird.

8. Verfahren nach Anspruch 5 oder 6, wobei die Zusammensetzung in Form eines Nahrungsmittels oder Getränks vorliegt.

## Revendications

1. Utilisation
i) d'une fraction de protéine basique dérivée du lait obtenue en adsorbant une protéine basique en mettant du lait ou une substance dérivée du lait en contact avec une résine échangeuse de cations et puis en éluant la fraction adsorbée sur la résine avec un éluant ayant une concentration de sel de 0,1 à 1 M;
et/ou
ii) d'une fraction de peptide basique obtenue par la dégradation de la fraction de protéine basique dérivée du lait avec une protéase
en tant que promoteur de production de collagène de la peau sous forme d'un cosmétique.

2. Utilisation selon la revendication 1, dans laquelle la fraction de protéine basique dérivée du lait est une fraction dont la composition en acides aminés contient 15% en poids ou plus d'un acide aminé basique.

3. Utilisation selon la revendication 1 ou 2, dans laquelle le promoteur de production de collagène de la peau est destiné à la prévention ou au traitement de rides de la peau, d'affaissements de la peau, de sensation de sécheresse de la peau ou de gerçures de la peau.

4. Utilisation
i) d'une fraction de protéine basique dérivée du lait obtenue en adsorbant une protéine basique en mettant du lait ou une substance dérivée du lait en contact avec une résine échangeuse de cations et puis en éluant la fraction adsorbée sur la résine avec un éluant ayant une concentration de sel de 0,1 à 1 M;
et/ou
ii) d'une fraction de peptide basique obtenue par la dégradation de la fraction de protéine basique dérivée du lait avec une protéase
en tant que promoteur de production de collagène de la peau sous forme d'un produit alimentaire ou d'une boisson.

5. Méthode cosmétique de traitement de la peau pour prévenir ou traiter des rides de la peau, des affaissements de la peau ou des gerçures de la peau, comprenant l'administration d'une composition comprenant une fraction de protéine basique dérivée du lait obtenue en adsorbant une protéine basique en mettant du lait ou une substance dérivée du lait en contact avec une résine échangeuse de cations et puis en éluant la fraction adsorbée sur la résine avec un éluant ayant une concentration de sel de 0,1 à 1 M.

6. Méthode cosmétique de traitement de la peau pour prévenir ou traiter des rides de la peau, des affaissements de la peau ou des gerçures de la peau, comprenant l'administration d'une composition comprenant une fraction de peptide basique dérivée du lait obtenue i) en adsorbant une protéine basique en mettant du lait ou une substance dérivée du lait en contact avec une résine échangeuse de cations et puis en éluant la fraction adsorbée sur la résine avec un éluant ayant une concentration de sel de 0,1 à 1 M, et ii) en dégradant ladite protéine basique avec une protéase.

7. Méthode selon la revendication 5 ou 6, dans laquelle la composition est sous forme d'un cosmétique à appliquer à la peau.

8. Méthode selon la revendication 5 ou 6, dans laquelle la composition est sous forme d'un produit alimentaire ou d'une boisson.
